# EUROPÄISCHE PATENTSCHRIFT

(11) **EP 1 469 005 B1**
(45) Veröffentlichungstag und Bekanntmachung des Hinweises auf die Patenterteilung: **30.08.2006**
(21) Anmeldenummer: 04001904.4
(22) Anmeldetag: 29.01.2004
(51) Int. Cl.: C07F 15/00, B01J 31/18, C07B 53/00

(54) **Verfahren zur asymmetrischen Hydrierung von Ketocarbonsäureestern**
Process for the asymmetric hydrogenation of ketocarbonic acid esters
Procédé d'hydrogénation asymétriques d' acides cétocaboniques

(30) Priorität: 12.02.2003 DE 10305943; 16.06.2003 DE 10326915
(43) Veröffentlichungstag der Anmeldung: 20.10.2004
(73) Patentinhaber: Saltigo GmbH, 51369 Leverkusen (DE)
(72) Erfinder: Thomas, John Meurig, Prof. Dr. Sir, Cambridge CB2 2PN (GB); Johnson, Brian F. G., Prof. Dr., Cambridge CB3 0DG (GB); Raja, Robert, Dr., Cambridge CB4 6DG (GB); Jones, Matthew David, Cambridge DB3 0HF (GB)
(74) Vertreter: Wichmann, Birgid

(56) Entgegenhaltungen:
- RAJA, ROBERT ET AL: "Constraining Asymmetric Organometallic Catalysts within Mesoporous Supports Boosts Their Enantioselectivity" JOURNAL OF THE AMERICAN CHEMICAL SOCIETY , 125(49), 14982-14983 CODEN: JACSAT; ISSN: 0002-7863, 15. November 2003 (2003-11-15), XP002286830
- DATABASE CHEMABS [Online] CHEMICAL ABSTRACTS SERVICE, COLUMBUS, OHIO, US; NINDAKOVA, L. O. ET AL: "Double stereoselection in hydrogenation of prochiral dehydrocarboxylic acids on Rh(S,S-DIODMA)2+TfO- complex in the presence of (+)-neomenthyldiphenylphosphine" XP002286833 gefunden im STN Database accession no. 2003:278863 & KHIMIYA V INTERESAKH USTOICHIVOGO RAZVITIYA , 11(1), 215-219 CODEN: KIURFI; ISSN: 0869-8538, 2003,
- TOMMASINO, M.L. ET AL.: "Cationic complexes with chiral diammine or dithiourea ligands as catalysts for molecular asymmetry hydrogenation" TETRAHEDRON ASYMMETRY, Bd. 10, 1999, Seiten 1813-1819, XP002286831
- ANN M ET AL: "RHODIUM AMINE COMPLEXES TETHERED ON SILICA-SUPPORTED GOLD-PALLADIUMBIMETAL CATALYSTS. ARENE HYDROGENATION" JOURNAL OF MOLECULAR CATALYSIS. A, CHEMICAL, ELSEVIER, AMSTERDAM, NL, Bd. 149, Nr. 1/2, 1999, Seiten 99-111, XP000923054 ISSN: 1381-1169
- JOHNSON B.F.G. ET AL.: "Superior Performance of Chiral Catalyst Confined with Mesoporous Silicia" CHEMICAL COMMUNICATIONS, 1999, Seiten 1167-1168, XP002286832

## Beschreibung

Die vorliegende Erfindung betrifft ein Verfahren zur Herstellung von enantiomerenangereicherten α- und β-Hydroxycarbonsäureestern aus den entsprechenden Ketocarbonsäureestern sowie dafür verwendbare Katalysatoren.

Enantiomerenangereicherte α- und β-Hydroxycarbonsäureester sind wertvolle Reagenzien zur Racematspaltung und wichtige Intermediate bei der Herstellung von Arzneimitteln und Agrochemikalien. Üblicherweise werden enantiomerenangereicherte α- und β-Hydroxycarbonsäureester durch katalytische Hydrierung der entsprechenden α- und β-Ketocarbonsäureester gewonnen, wobei als Katalysatoren häufig Übergangsmetallkomplexe mit chiralen Phosphanen als Liganden eingesetzt werden (siehe z.B. Genet et al., Tetrahedron, Asymmetry, 1994, 5(4), 675-690). Nachteilig an chiralen Phosphanen ist der hoher Preis und die Oxidationsempfindlichkeit weshalb ihr Einsatz im industriellen Maßstab wenn überhaupt überwiegend in homogenen Prozessen erfolgt.

Alternativ dazu sind Verfahren unter Verwendung von mit Cinchona-Alkaloiden oder Weinsäurederivaten modifizierten Platin- oder Nickelkatalysatoren bekannt (T. Mallat et al., Fine Chemicals through Heterogeneous Catalysis, Wiley-VCH, 2001, S. 449 ff).

Darüberhinaus wird für die Hydrierung von Ketoestern in Ferrand et al. (Tetrahedron: Asymmetry, 13, 2002, S. 1379 bis 1384) der Einsatz von Rhodium-, Ruthenium- und Iridium-Komplexen mit chiralen Diaminen beschrieben. Allen Verfahren ist jedoch gemeinsam, dass sie allenfalls einen mäßigen Enantiomerenüberschuss erlauben.

Es bestand daher das Bedürfnis, Katalysatoren bereitzustellen, die insbesondere in einem Verfahren zur Herstellung von enantiomerenangereicherten α- und β-Hydroxycarbonsäureestern hohe Ausbeuten und Enantioselektivitäten ermöglichen.

Es wurden nun Substanzen gefunden,
enthaltend zumindest
- ein mikro-, meso- oder makroporöses Trägermaterial und
- daran und/oder darin adsorbierte Verbindungen der Formel (I) 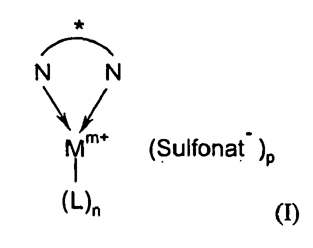 in der 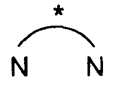 für eine enantiomerenangereicherte chirale Stickstoffverbindung steht,
   - (M ^{m+}): für ein Metall mit der Wertigkeit m steht
   - L: für einen anionischen oder neutralen Liganden steht
   - (Sulfonat⁻): für das Anion einer Sulfonsäure steht und
   - p: für eins oder zwei steht und
   - n: für eins, zwei, drei oder vier steht,
   wobei die Auflage gilt, dass m-p-[Anzahl der anionischen Liganden] = 0 ist.

Enantiomerenangereicherte Verbindungen im Sinne der Erfindung sind enantiomerenreine Verbindungen oder Mischungen von Enantiomeren einer Verbindung, in denen ein Enantiomer in einem Enantiomerenüberschuss, im Folgenden auch ee (enantiomeric excess) genannt, im Vergleich zum anderen Enantiomer vorliegt. Bevorzugt beträgt dieser Enantiomerenüberschuss 10 bis 100 % ee, besonders bevorzugt 90 bis 100 % ee und ganz besonders bevorzugt 95 bis 100 % ee.

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.

Im Folgenden stehen **Alkyl** beziehungsweise **Alkoxy** beziehungsweise **Alkylen** beziehungsweise **Alkenylen** jeweils unabhängig für einen geradkettigen, cyclischen, verzweigten oder unverzweigten Alkyl- beziehungsweise Alkoxy- beziehungsweise Alkylen- beziehungsweise Alkenylen-Rest, der gegebenenfalls weiter durch C₁-C₄-Alkoxy substituiert sein kann. Gleiches gilt für den nichtaromatischen Teil eines Arylalkyl-Restes.

C₁-C₄-Alkyl steht beispielsweise für Methyl, Ethyl, n-Propyl, iso-Propyl, n-Butyl, sec.-Butyl und tert.-Butyl, C₁-C₈-Alkyl darüber hinaus beispielsweise für n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, 1-Ethyl-2-methylpropyl, n-Heptyl und n-Octyl, C₁-C₂₀-Alkyl weiter darüber hinaus beispielsweise für Adamantyl, die isomeren Menthyle, n-Nonyl, n-Decyl und n-Dodecyl.

C₁-C₄-Alkoxy steht beispielsweise für Methoxy, Ethoxy, n-Propoxy, iso-Propoxy, n-Butoxy, sec.-Butoxy und tert.-Butoxy, C₁-C₈-Alkoxy darüberhinaus beispielsweise für n-Pentoxy, 1-Methylbutoxy, 2-Methylbutoxy, 3-Methylbutoxy, neo-Pentoxy, 1-Ethylpropoxy, cyclo-Hexoxy, cyclo-Pentoxy, n-Hexoxy und n-Octoxy, C₁-C₂₀-Alkoxy weiter darüber hinaus beispielsweise für Adamantoxy, die isomeren Menthoxy-Reste, n-Decoxy und n-Dodecoxy.

C₁-C₄-Alkylen steht beispielsweise für Methylen, 1,1-Ethylen, 1,2-Ethylen, 1,1-Propylen, 1,3-Propylen, 1,4-Butylen, C₁-C₈-Alkylen darüber hinaus beispielsweise für 1,2-cyclo-Hexylen und 1,2-cyclo-Pentylen.

C₂-C₈-Alkenylen steht beispielsweise für 1,1-Ethenylen 2-Ethoxy-1,1-ethenylen und 2-Methoxy-1,1-ethenylen.

**Halogenalkyl,** beziehungsweise **Halogenalkoxy,** beziehungsweise **Halogenalkylen** bedeutet jeweils unabhängig einen geradkettigen, cyclischen, verzweigten oder unverzweigten Alkyl-Rest beziehungsweise Alkylen-Rest, der einfach, mehrfach oder vollständig durch Halogenatome substituiert ist.

Beispielsweise steht C₁-C₂₀-Halogenalkyl für Trifluormethyl, Chlormethyl, 2-Chlorethyl, 2,2,2-Trifluorethyl, Pentafluorethyl, Nonafluorbutyl, Heptafluorisopropyl, Perfluoroctyl, Perfluordodecyl und Perfluorhexadecyl.

Aryl steht jeweils unabhängig für einen heteroaromatischen Rest mit 5 bis 14 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, oder und vorzugsweise für einen carbocyclischen aromatischen Rest mit 6 bis 14 Gerüstkohlenstoffatomen.

Beispiele für carbocyclische aromatische Reste mit 6 bis 14 Gerüstkohlenstoffatomen sind zum Beispiel Phenyl, Biphenyl, Naphtyl, Phenanthrenyl, Anthracenyl oder Fluorenyl, heteroaromatische Reste mit 5 bis 14 Gerüstkohlenstoffatomen in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, sind beispielsweise Pyridinyl, Oxazolyl, Benzofuranyl, Dibenzofuran-yl oder Chinolinyl.

Weiterhin kann der carbocyclische aromatische Rest oder heteroaromatische Rest mit bis zu fünf gleichen oder verschiedenen Substituenten pro Cyclus substituiert sein, die beispielsweise ausgewählt sind aus der Gruppe Nitro, Cyano, Chlor, Fluor, C₁-C₁₂-Alkyl, C₁-C₁₂-Halogenalkyl, C₁-C₁₂-Halogenalkoxy, C₁-C₁₂-Halogenalkylthio, C₁-C₁₂-Alkoxy, Di(C₁-C₈-alkyl)amino oder Tri(C₁-C₆-alkyl)siloxyl substituiert sein.

**Arylen** steht für einen Aryl-Rest, der eine weitere Bindungsstelle am aromatischen Gerüst besitzt und dadurch divalent ist.

**Arylalkyl** bedeutet jeweils unabhängig einen geradkettigen, cyclischen, verzweigten oder unverzweigten Alkyl-Rest nach vorstehender Definition, der einfach, mehrfach oder vollständig durch Aryl-Reste gemäß vorstehender Definition substituiert sein kann.

**Arylalkylen** steht für einen Arylalkyl-Rest, der eine weitere Bindungsstelle am aromatischen Gerüst besitzt und dadurch divalent ist.

Im Folgenden werden Vorzugsbereiche für die erfindungsgemäßen Substanzen definiert:

Bevorzugte **Trägermaterialien** besitzen eine Porengröße, die im Bereich von 15 bis 250 Å liegt, besonders bevorzugt im Bereich von 20 bis 100 Å. Die im Rahmen der Erfindung geltenden Definitionen für die Begriffe mikro-, meso- und makroporös wie auch die Nomenklatur der Zeolithe sind dabei IUPAC-konform auszulegen (McCusker et al. Pure Appl. Chem, vol. 73, No. 2, pp 381-394, 2001). Geeignete Trägermaterialien sind beispielsweise Silica-Gele, Zeolithe vom Typ Davison, MOR, X, Y, MCM, ZSM, FAU, MFI, L, BEA, FER, A und SBA, sowie solche vom Typ AlPO, MAlPO und SAPO, wobei die genannten Zeolithe gegebenenfalls isomorph substituiert sein können. Besonders bevorzugt sind Trägermaterialien wie insbesondere solche des MCM- oder Davison-Typs wie beispielsweise MCM-41 (ca. 30 Å), Davison 923 (ca. 22 Å), Davison 634 (ca. 60 Å).
In Formel (I) steht 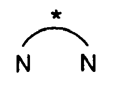 bevorzugt für enantiomerenangereicherte chirale Stickstoffverbindungen der Formel (II) 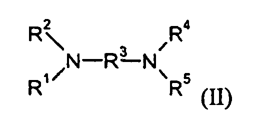 in der
R¹, R², R⁴ und R⁵ jeweils unabhängig voneinander für Wasserstoff, C₁-C₈-Alkyl, C₅-C₁₅-Arylalkyl oder C₄-C₁₄-Aryl stehen oder NR¹R² und/oder NR⁴R⁵ als Ganzes für einen cyclischen Aminorest mit insgesamt 4 bis 20 Kohlenstoffatomen steht,
R³ für einen divalenten Rest mit insgesamt 2 bis 30 Kohlenstoffatomen stehtoder
R³ und mindestens einer der Reste R¹, R², R⁴, R⁵ zusammen Teil eines cyclischen Aminorestes mit insgesamt 4 bis 20 Kohlenstoffatomen sind.

Bevorzugte Verbindungen der Formel (II) sind solche, in der
R¹, R², R⁴ und R⁵ jeweils unabhängig voneinander für Wasserstoff, C₁-C₈-Alkyl, C₅-C₁₅-Arylalkyl oder C₄-C₁₄-Aryl stehen oder NR¹R² und/oder NR⁴R⁵ als Ganzes für einen 5- oder 6-gliedrigen monocyclischen Aminorest steht, der am Kohlenstoffgerüst gegebenenfalls einfach, zweifach, dreifach oder vierfach C₁-C₄-Alkyl substituiert ist und
R³ für einen divalenten Rest steht, der ausgewählt ist aus der Gruppe C₂-C₈-Alkylen, das gegebenenfalls einfach oder zweifach durch C₄-C₁₄-Arylreste weiter substituiert sein kann, C₅-C₁₅-Arylalkylen, C₄-C₁₄-Arylen oder Bis-(C₄-C₁₄-arylen) oder
R³ und einer der Reste R¹, R², R⁴ und R⁵ zusammen Teil eines 5- oder 6-gliedrigen monocyclischen Aminorestes sind, der am Kohlenstoffgerüst gegebenenfalls zusätzlich einfach, zweifach, dreifach oder vierfach durch C₁-C₄-Alkyl substituiert ist.

Besonders bevorzugte Verbindungen der Formel (II) sind solche, in denen
R¹, R², R⁴ und R⁵ jeweils unabhängig voneinander für Wasserstoff, Methyl oder Ethyl stehen und
R³ für einen divalenten Rest steht, der ausgewählt ist aus der Gruppe 1,2-Bis-(C₄-C₁₄-aryl)-1,2-ethylen, 1,2-Cyclohexylen, 1,1'-2,2'-Bis-(C₄-C₁₄-arylen) oder
R³ und einer der Reste R¹, R², R⁴ und R⁵ zusammen Teil eines Pyrrolidinyl- oder Piperidinyl-Restes sind.

Ganz besonders bevorzugte Verbindungen der Formel (II) sind
(1R,2R)-1,2-Diphenylethylendiamin, (1S,2S)-1,2-Diphenylethylendiamin, (1R,2R)-1,2-Dimethylethylendiamin, (1S,2S)-1,2-Dimethylethylendiamin, (1R,2R)-1,2-cyclohexylendiamin, (1S,2S)-1,2-cyclohexylendiamin, (S)-2-Aminomethyl-1-ethylpyrrolidin, (R)-2-Aminomethyl-1-ethyl-pyrrolidin; (S)-(2-Pyrrolidinylmethyl)-pyrrolidin, (R)-(2-Pyrrolidinylmethyl)-pyrrolidin, (S)-2-Aminomethyl-1-methylpyrrolidin, (R)-2-Aminomethyl-1-methylpyrrolidin, (R)-1,1'-Diamino-2,2'-binaphthyl, (S)-1,1'-Diamino-2,2'-binaphthyl, (R)-1,1'-Diamino-6,6'-Dimethoxy-2,2'-biphenyl und (S)-1,1'-Diamino-6,6'-Dimethoxy-2,2'-biphenyl, wobei (R)-2-Aminomethyl-1-ethylpyrrolidin, (S)-(2-Pyrrolidinylmethyl)-pyrrolidin, (R)-(2-Pyrrolidinylmethyl)-pyrrolidin und (S)-2-Aminomethyl-1-methylpyrrolidin noch weiter bevorzugt sind.

In Formel (I) steht weiterhin
- (M ^{m+}): vorzugsweise für Cobalt in den formalen Oxidationsstufen 0, +2 und +3, Rhodium und Iridium in den formalen Oxidationsstufen +1 und +3, Nickel, Palladium und Platin in den formalen Oxidationsstufen 0 und +2 sowie Ruthenium in der formalen Oxidationsstufe +2, wobei Rh^{I}, Ir^{I} und Pd^{II} bevorzugt sind.
- L: steht bevorzugt für folgende Ligandentypen: Monoolefine wie beispielsweise Ethylen, Cycloocten und Cyclohexen, Diolefine wie beispielsweise 1,5-Cyclooctadien (cod), Norbomadien (nbd), und Butadien, Nitrile wie Acetonitril (ACN), Benzonitril und Benzylnitril, Aromaten wie Benzol, Mesitylen und Cymol, sowie anionische Liganden wie Allyl, Methylallyl, Phenylallyl, C₁-C₈₋Alkylacylacetonate, C₁-C₈-Alkylacylate, Chlorid, Bromid und Iodid.
- (Sulfonat⁻): steht bevorzugt für Salze des Typs R⁶SO₃⁻, wobei R⁶ für C₁-C₁₂-Alkyl, C₁-C₂₀₋Halogenalkyl, C₄-C₁₄-Aryl oder C₅-C₁₅-Arylalkyl steht. Bevorzugt steht R⁶ für Methyl, Phenyl, p-Tolyl und C₁-C₂₀-Perfluoralkyl, besonders bevorzugt für C₁-C₄₋Perfluoralkyl wie insbesondere Trifluormethyl.

Besonders bevorzugt steht 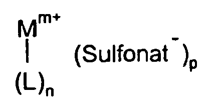 als ganzes Fragment für Rh(cod)OTf, Ir(cod)OTf, Rh(nbd)OTf, Ir(nbd)OTf, Pd(Allyl)OTf, Rh(cod)OMes, Ir(cod)OMes, Rh(nbd)OMes, Ir(nbd)OMes, Pd(Allyl)OMes, Rh(cod)ONf, Ir(cod)ONf, Rh(nbd)ONf, Ir(nbd)Onf und Pd(Allyl)ONf, wobei OTf für Trifluormethansulfonat, OMes für Methansulfonat und ONf für Nonafluorbutansulfonat steht.

Ganz besonders bevorzugte Verbindungen der Formel (I) sind solche der Formeln (Ia), (Ib), (Ic), (Id), (Ie) und (If) 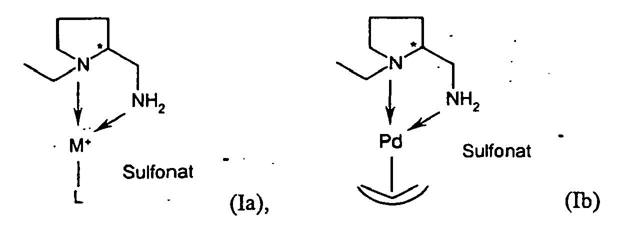 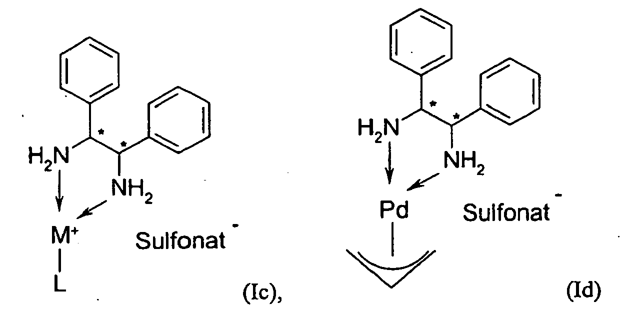 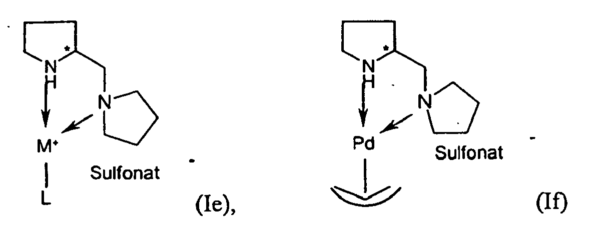 in denen jeweils
- *: ein stereogenes Zentrum markiert, das entweder R oder S konfiguriert ist, wobei die Auflage gilt, dass Mesoformen ausgeschlossen sind (Verbindungen der Formel (Ic) und (Id))
- M⁺: für Rhodium^{I} oder Iridium^{I} steht und
- L: für cod oder nbd steht und
- Sulfonat⁻: für Trifluormethansulfonat, Mesylat oder Nonafluorbutansulfonat steht.

Die Verbindungen der Formel (I) sind von der Erfindung ebenfalls umfasst, wobei folgende ausgenommen sind:
[Rh(cod)((S)-2-aminomethyl-1-ethylpyrrolidin)]OTf und [Rh(cod)((1R,2R)-1,2-Diphenylethylen-diamin)]OTf.

Die Herstellung der Verbindungen der Formel (I) wie insbesondere solchen der Formeln (Ia) bis (If) kann in an sich bekannter Weise beispielsweise dadurch erfolgen, dass enantiomerenangereicherte chirale Stickstoffverbindungen der Formel (II) vorzugsweise in Gegenwart eines organischen Lösungsmittels mit Übergangsmetallverbindungen umgesetzt werden.

Als organische Lösungsmittel für die Umsetzung eignen sich üblicherweise aliphatische oder aromatische, gegebenenfalls halogenierte Kohlenwasserstoffe, wie beispielsweise Petrolether, Benzol, Toluol, die isomeren Xylole, Chlorbenzol, die isomeren Dichlorbenzole, Hexan, Cyclohexan, Dichlormethan oder Chloroform sowie vorzugsweise Ether, wie Diethylether, Diisopropylether, Dioxan, Tetrahydrofuran, Methyl-tert.-butylether oder Ethylenglykoldimethyl- oder -diethylether. Besonders bevorzugte organische Lösungsmittel sind Toluol, Diethylether, Tetrahydrofuran und Methyl-tert.-butylether.

Bevorzugte Übergangsmetallverbindungen für die Umsetzung mit enantiomerenangereicherten chiralen Stickstoffverbindungen der Formel (II) sind solche der Formel (IIIa)

M¹(An¹)ₚ₁ (IIIa)

in der
- M¹: für Ruthenium, Rhodium, Iridium, Nickel, Palladium oder Platin und
- An¹: für Halogenid und
- p1: für Ruthenium, Rhodium und Iridium für 3, für
Nickel, Palladium und Platin für 2 steht,
oder Übergangsmetallverbindungen der Formel (IIIb)

M²(An²)ₚ₂L¹₂ (IIIb)

in der
- M²: für Ruthenium, Rhodium, Iridium, Nickel, Palladium oder Platin und
- An²: für Halogenid oder ein Sulfonat
- p2: für Rhodium und Iridium für 1, für
Nickel, Palladium, Platin und Ruthenium für 2 steht und
- L¹: jeweils für ein C₂-C₁₂-Alken wie beispielsweise Ethylen oder Cycloocten, oder ein Nitril wie beispielsweise Acetonitril, Benzonitril oder Benzylnitril steht, oder
- L¹₂: zusammen für ein (C₄-C₁₂)-Dien wie beispielsweise Norbomadien oder 1,5-Cyclooctadien steht
oder Übergangsmetallverbindungen der Formel (IIIc)

[M³L²An³₂]₂ (IIIc)

in der
- M³: für Ruthenium und
- L²: für cod, nbd, Allyl, Methylallyl oder Arylreste wie zum Beispiel Cymol, Mesitylen, Benzol und
- An³: für Halogenid oder Sulfonat steht
oder Übergangsmetallverbindungen der Formel (IIId)

M⁴ₚ₃[M⁵(An³)₄] (IIId),

wobei
- M⁵: für Palladium, Nickel, Iridium oder Rhodium und
- An³: für Chlorid oder Bromid steht und
- M⁴: für Lithium, Natrium, Kalium, Ammonium oder organisches Ammonium steht und
- p3: für Rhodium und Iridium für 3, für
Nickel, Palladium und Platin für 2 steht,
oder Übergangsmetallverbindungen der Formel (IIIe)

[M⁶(L³)₂]An⁴ (IIIe),

wobei
- M⁶: für Iridium oder Rhodium und
- L³: für ein (C₄-C₁₂)-Dien wie beispielsweise Norbomadien oder 1,5-Cyclooctadien steht und
- An⁴: für ein Sulfonat steht.

Darüber hinaus sind als Übergangsmetallverbindungen beispielsweise Ni(cod)₂, Pd₂(dibenzylidenaceton)₃, Cyclopentadienyl₂Ru, Rh(acetylacetonat)(CO)₂, Ir(pyridin)₂(cod) oder mehrkernige verbrückte Komplexe wie beispielsweise [Pd(Allyl)Cl]₂, [Pd(Allyl)Br]₂, [Rh(cod)Cl]₂, [Rh(cod)Br]₂, [Rh(Ethen)₂Cl]₂, [Rh(Cycloocten)₂Cl]₂, [Ir(cod)Cl]₂ und [Ir(cod)Br]₂, [Ir(Ethen)₂Cl]₂ und [Ir(Cycloocten)₂Cl]₂ geeignet.

Besonders bevorzugte Übergangsmetallverbindungen sind: [Pd(Allyl)Cl]₂, [Pd(Allyl)Br]₂, [Rh(cod)Cl]₂, [Rh(cod)₂Br, [Rh(cod)₂]OTf, [Rh(cod)₂]OMes, [Rh(cod)₂]ONf, RuCl₂(cod), [(Cymol)RuCl₂]₂, [(Benzol)RuCl₂]₂, [(Mesitylen)RuCl₂]₂, [(Cymol)RuBr₂]₂, [(Cymol)RuI₂]₂, [Ir(cod)₂Cl]₂, [Ir(cod)₂]OTf, [Ir(cod)₂]OMes, [Ir(cod)₂]Onf, [Rh(nbd)₂Br], [Rh(nbd)₂]OTf, [Rh(nbd)₂]OMes, [Rh(nbd)₂]Onf, RuCl₂(nbd), [Ir(nbd)₂]OTf, [Ir(nbd)₂]OMes, [Ir(nbd)₂]ONf, Ir(pyridin)₂(nbd)OTf, [Ru(DMSO)₄Cl₂], [Ru(ACN)₄Cl₂], [Ru(PhCN)₄Cl₂] und [Ru(cod)Cl₂]ₙ.

Es sei darauf hingewiesen, dass es bei Verwendung von halogenidhaltigen Übergangsmetallverbindungen erforderlich ist, zusätzlich in etwa äquimolarer Menge zum vorhandenen Halogenid beispielsweise Thallium-, Silber- oder Kalium-Sulfonate gemäß oben stehender

Definition einzusetzen.

Zur Herstellung der erfindungsgemäßen Substanzen wird das Trägermaterial mit Verbindungen der Formel (I) umgesetzt.

Das Gewichtsverhältnis von Verbindungen der Formel (I) zu Trägermaterial kann dabei beispielsweise und bevorzugt 0,02:1 bis 100:1, besonders bevorzugt 0,1:1 bis 5:1 und ganz besonders bevorzugt 0,1:1 bis 1:1 betragen.

Die Reaktionstemperatur kann beispielsweise und bevorzugt -20 bis 100°C, besonders bevorzugt 0 bis 80°C und ganz besonders bevorzugt 10 bis 30°C betragen.

Die Aufarbeitung der erfindungsgemäßen Substanzen kann in an sich bekannter Weise durch Filtration und/oder Zentrifugation und/oder Sedimentation und gegebenenfalls anschließendem Waschen mit organischem Lösungsmittel erfolgen, wobei das Waschen beispielsweise diskontinuierlich oder kontinuierlich durchgeführt werden kann. Zu Lagerzwecken werden die erfindungsgemäßen Verbindungen vorzugsweise getrocknet.

Die erfindungsgemäßen Substanzen können direkt als Katalysator für asymmetrische Reaktionen eingesetzt werden.

Von der Erfindung sind daher auch Katalysatoren umfasst, die die erfindungsgemäßen Substanzen enthalten.

Weiterhin ist von der Erfindung ein Verfahren zur katalytischen Herstellung von enantiomerenangereicherten Verbindungen umfasst, das dadurch gekennzeichnet ist, dass als Katalysatoren solche eingesetzt werden, die erfindungsgemäßen Substanzen enthalten.

Bevorzugte Verfahren zur Herstellung von enantiomerenangereicherten Verbindungen sind asymmetrische Hydrierungen, wie beispielsweise Hydrierungen von prochiralen C=C-Bindungen wie prochiralen Enaminen, Olefinen, Enolethem; C=O-Bindungen wie prochiralen Ketonen und C=N-Bindungen wie prochiralen Iminen. Besonders bevorzugte asymmetrische Hydrierungen sind Hydrierungen von prochiralen Ketonen wie insbesondere α- und β-Ketocarbonsäureestern.

Bevorzugte α- und β-Ketocarbonsäureester sind Verbindungen der Formel (IV), 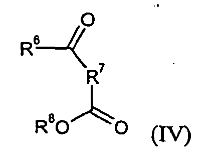 in der
R⁶ und R⁸ jeweils unabhängig voneinander für C₁-C₁₂-Alkyl, C₁-C₁₂-Halogenalkyl, C₅-C₁₅₋Arylalkyl oder C₄-C₁₄-Aryl stehen und
R⁷ fehlt oder für 1,1-(C₁-C₄-Alkylen) steht.

Vorzugsweise stehen
R⁶ und R⁸ jeweils unabhängig voneinander für gegebenenfalls chloriertes C₁-C₄-Alkyl oder Phenyl und
R⁷ für Methylen oder fehlt.

Besonders bevorzugte Verbindungen der Formel (IV) sind Phenylglyoxylsäuremethylester, Benzoylameisensäuremethylester und Chloracetessigester.

Durch erfindungsgemäße Hydrierung von α- und β-Ketocarbonsäureestern sind enantiomerenangereicherte Verbindungen der Formel (V) erhältlich, 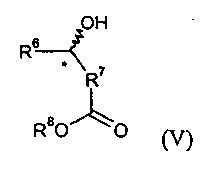 in der
* ein stereogenes Zentrum markiert, das S oder R-konfiguriert ist und
R⁵, R⁶ und R⁷ die unter der Formel (V) angegebenen Bedeutungen und Vorzugsbereiche besitzen.

In einer bevorzugten Ausführungsform von erfindungsgemäßen asymmetrischen Hydrierungen beträgt die Reaktionstemperatur 0 bis 200°C, bevorzugt 10 bis 150°C, der Wasserstoffpartialdruck beispielsweise 0,1 bis 200 bar, bevorzugt 0,9 bis 100 bar und besonders bevorzugt 4 bis 30 bar.

Als Lösungsmittel für erfindungsgemäße asymmetrische Hydrierungen eignen sich insbesondere aliphatische oder aromatische, gegebenenfalls halogenierte Kohlenwasserstoffe, wie beispielsweise Petrolether, Benzol, Toluol, die isomeren Xylole, Chlorbenzol, die isomeren Dichlorbenzole, Hexan, Cyclohexan, Dichlormethan oder Chloroform, Ether, wie Diethylether, Diisopropylether, Dioxan, Tetrahydrofuran, Methyl-tert.-butylether oder Ethylenglykoldimethyl-oder -diethylether sowie vorzugsweise Alkohole wie Methanol, Ethanol und Iso-Propanol.

Das Gewichtsverhältnis der erfindungsgemäßen Katalysatoren zu Substrat kann beispielsweise 1 : 1 bis 1:10000 betragen, bevorzugt ist ein Verhältnis von 1:5 bis 1:1000.

Der Vorteil der vorliegenden Erfindung ist, dass auf effiziente Weise heterogene Katalysatoren in hohen Ausbeuten hergestellt werden können und diese Katalysatoren hohe Umsätze und Enantioselektivitäten in asymmetrischen Synthesen erlauben. Dieser Umstand ist insofern als besonders überraschend einzustufen, dass die Verbindungen der Formel (I) im Falle des homogenen Einsatzes wenn überhaupt nur sehr geringe Enantioselektivitäten erlauben, wie die Vergleichsbeispiele zeigen.

### Beispiele

### Beispiel 1

### Herstellung von [Rh(cod)((S)-2-Aminomethyl-1-ethylpyrrolidin)]CF₃SO₃

[Rhcl(cod]₂ (100 mg, 0.20 mmol) wurde in THF (10 ml) gelöst, AgCF₃SO₃ (104 mg, 0.40 mmol) zugegeben und die Lösung für eine Stunde gerührt. Die Lösung wurde anschließend filtriert, das Filtrat mit (S)-2-Aminomethyl-1-ethylpyrrolidin (52 mg, 0.40 mmol) versetzt und die resultierende Lösung eine Stunde gerührt. Anschließend wurde die Lösung im Vakuum eingeengt und mit Hexan (25 ml) versetzt, wobei das Produkt ausfiel. Es wurde filtriert, das Produkt mit Hexan (2 x 20 ml) und Diethylether (2 x 20 ml) gewaschen und im Vakuum getrocknet. Es wurde ein gelbes Pulver erhalten (172 mg, 88 %).

Anal.: Berechnet für C₁₅H₂₈N₂RhBF₄ C, 39.34; H, 5.74; N, 5.74. Gefunden: C, 39.84; H, 5.54; N, 5.69.
¹H NMR (CDCl₃) 1.76-4.3 (28H, Amin und Olefin).
¹³C NMR (CDCl₃) = 12.2 (1), 21.7 (4), 24.4 (5), 45.5 (7), 51.0 (2), 56.5 (3), 67.1 (6), 30.4, 30.7 (CH₂) 79.7, 83.6 (CH).
+ve ESI = 339 (M⁺).

### Beispiel 2

### Herstellung von heterogenisiertem [Rh(cod)((S)-2-Aminomethyl-1-ethyl-pyrrolidin)]CF₃SO₃

Zu trockenem calciniertem MCM-41 (500mg) und CH₂Cl₂ (20 ml) wurde der Komplex aus Beispiel 1 gegeben. Es wurde drei Stunden gerührt. In dieser Zeit färbte sich der Träger MCM-41 gelb. Anschließend wurde filtriert, der Rückstand mit reichlich CH₂Cl₂ gewaschen bis kein Komplex mehr erkennbar ausgewaschen wurde und das Produkt im Vakuum getrocknet.

Anal: C, 3.77; H, 0.83; N, 0.42.

### Beispiel 3

### Herstellung von [Rh(cod)((1R,2R)-1,2-Diphenylethylendiamin)]CF₃SO₃

[RhCl(cod]₂ (100 mg, 0.20 mmol) wurde in THF (10 ml) gelöst, AgCF₃SO₃ (104 mg, 0.40 mmol) zugegeben und die Lösung für eine Stunde gerührt. Die Lösung wurde anschließend filtriert, das Filtrat mit (1R, 2R)-1,2-Diphenylethylendiamin (80 mg, 0.4 mmol) versetzt und die resultierende Lösung eine Stunde gerührt. Anschließend wurde die Lösung im Vakuum eingeengt und mit Hexan (25 ml) versetzt, wobei das Produkt ausfiel. Es wurde filtriert, das Produkt mit Hexan (2 x 20 ml) und Diethylether (2 x 20 ml) gewaschen und im Vakuum getrocknet. Es wurde ein gelbes Pulver erhalten (200 mg, 88 %).
Anal.: Berechnet für C₂₃H₂₈N₂RhF₃SO₃ C, 48.25; H, 4.90; N, 4.90. Gefunden: C, 47.95; H, 4.86; N, 4.60.
¹H NMR (CD₃OD) 1.95 (br m, C*H*₂ 4H), 2.45 (br m, C*H*₂, 4H), 4.01 (s, NC*H*, 2H), 4.23 (m, C*H*, 2H), 4.35 (m, C*H*, 2H), 7.1-7.3 (m, Ph, 10H).
¹³C NMR (CD₃OD) 31.5 (*C*H₂), 66.3 (N*C*H) 81.4 (*C*H), 128.5, 129.2, 129.68, 140.5 (Ph).
+ve ESI = 423 (M⁺).

### Beispiel 4

### Herstellung von heterogenisiertem [Rh(cod)((1R,2R)-1,2-Diphenylethylendiamin)]CF₃SO₃

Zu trockenem calciniertem MCM-41 (500mg) und CH₂Cl₂ (20 ml) wurde der Komplex aus Beispiel 3 gegeben. Es wurde drei Stunden gerührt. In dieser Zeit färbte sich der träger MCM-41 gelb. Anschließend wurde filtriert, der Rückstand mit reichlich CH₂Cl₂ gewaschen bis kein Komplex mehr erkennbar ausgewaschen wurde und das Produkt im Vakuum getrocknet.

Anal: C, 3.76; H, 0.72; N, 0.39.

### Beispiele 5 und 6

**Analog zu Beispiel 3 wurden erhalten:**
5) [Rh(cod)((S)-(2-Pyrrolidinmethyl-)-pyrrolidin)]CF₃SO₃
6) [Pd(allyl)((S)-(2-Pyrrolidinmethyl-)-pyrrolidin)]CF₃SO₃

### Beispiele 7-16

**Analog zu Beispiel 4 wurden erhalten:**
7) [Rh(cod)((1R,2R)-1,2-Diphenylethylendiamin)]CF₃SO₃ auf/in Davison 923
8) [Rh(cod)((1R,2R)-1,2-Diphenylethylendiamin)]CF₃SO₃ auf/in Davison 634
9) [Rh(cod)((1R,2R)-1,2-Diphenylethylendiamin)]CF₃SO₃ auf/in Davison 654
10) [Rh(cod)((S)-(2-Pyrrolidinmethyl-)-pyrrolidin)]CF₃SO₃ auf/in Davison 923
11) [Rh(cod)((S)-(2-Pyrrolidinmethyl-)-pyrrolidin)]CF₃SO₃ auf/in Davison 634
12) [Rh(cod)((S)-(2-Pyrrolidinmethyl-)-pyrrolidin)]CF₃SO₃ auf/in Davison 654

**Analog zu Beispiel 2 wurden** erhalten:
13) [Rh(cod)((S)-2-Aminomethyl-1-ethyl-pyrrolidin)]CF₃SO₃ auf/in Davison 923
14) [Rh(cod)((S)-2-Aminomethyl-1-ethyl-pyrrolidin)]CF₃SO₃ auf/in Davison 634
15) [Rh(cod)((S)-2-Aminomethyl-1-ethyl-pyrrolidin)]CF₃SO₃ auf/in Davison 653
16) [Pd(allyl)((S)-(2-Pyrrolidinmethyl-)-pyrrolidin)]CF₃SO₃ auf/in MCM 41

### Beispiele 17 bis 44: Asymmetrischen Hydrierungen

### Allgemeine Arbeitsvörschrift

Die asymmetrischen Hydrierungen wurden in einem Hochdruckautoklaven aus rostfreiem Edelstahl mit einem Volumen von 150 ml durchgeführt. Jeweils 10 mg des homogenen Katalysators bzw. jeweils 50 mg der immoblilisierten Katalysatoren wurden unter Inertatmosphäre in den Hochdruckautoklaven transferiert.

Das Substrat (0.5 g), Methanol (30 g), und ein interner Standard (Cyclododecan) wurden zugegeben und der Hochdruckautoklav verschlossen. Der Hochdruckautoklav und seine Zu- und Ableitungen wurden anschließend durch dreimaliges Spülen mit Stickstoff inertisiert und zur Prüfung der Dichtigkeit schließlich unter einen Wasserstoffdruck von 5 bar gesetzt. Anschließend wurde der Wasserstoffdruck auf 20 bar erhöht, der Hochdruckautoklav auf Reaktionstemperatur gebracht (313 K) und der Inhalt mit einem mechanischen Rührer bei 400 U/min gerührt.

Über ein automatisches Entnahmeventil wurden Proben des Inhalts entnommen um den Verlauf der Reaktion untersuchen zu können. Am Ende der Reaktion wurde der Hochdruckautoklav zwei Stunden im Eisbad gekühlt, entspannt und die Produkte durch Gaschromatographie (GC, Varian, Model 3400 CX) über eine chirale Säule identifiziert (Chiraldex, 20 m x 0.25 mm).

Die Ergebnisse der Hydrierungsexperimente sind in nachstehender Tabellen zusammengefasst:

**Substrat : Benzoylameisensäuremethylester**

| Substrat: Benzoylameisensäuremethylester | | | | | | |
|---|---|---|---|---|---|---|
| **Beispiel** | **Katalysator aus Beispiel** | **Reaktionstyp** | **t (h)** | **Umsatz (%)** | **TOF (h**^{**-1**}**)** | **ee (%)** |
| **17** | 5 | Homogen | 0.5 | 46.2 | 145 | 53 |
| **18** | 10 | Heterogen | 0.5 | 92.8 | 643 | 85 |
| **19** | 10 | Heterogen | 2.0 | 95.8 | 166 | 94 |
| **20** | 11 | Heterogen | 0.5 | 63.0 | 436 | 72 |
| **21** | 11 | Heterogen | 2.0 | 91.5 | 159 | 78 |
| **22** | 12 | Heterogen | 0.5 | 60.7 | 420 | 65 |
| **23** | 12 | Heterogen | 2.0 | 86.9 | 151 | 59 |
| **24** | 1 | Homogen | 2.0 | 62 | 46 | 0 |
| **25** | 13 | Heterogen | 0.5 | 82.6 | 542 | 82 |
| **26** | 13 | Heterogen | 2.0 | 93.3 | 153 | 77 |
| **27** | 14 | Heterogen | 0.5 | 67.1 | 440 | 65 |
| **28** | 14 | Heterogen | 2.0 | 93.9 | 154 | 61 |
| **29** | 15 | Heterogen | 0.5 | 44.6 | 292 | 0 |
| **30** | 15 | Heterogen | 2.0 | 86.1 | 141 | 0 |
| **31** | 3 | Homogen | 2.0 | 69.9 | 60 | 0 |
| **32** | 7 | Heterogen | 0.5 | 77.7 | 596 | 50 |
| **33** | 7 | Heterogen | 2.0 | 98.1 | 188 | 79 |
| **34** | 8 | Heterogen | 0.5 | 59.7 | 458 | 68 |
| **35** | 8 | Heterogen | 1.0 | 75.5 | 290 | 73 |
| **36** | 9 | Heterogen | 0.5 | 38.8 | 298 | 0 |
| **37** | 9 | Heterogen | 2.0 | 83.1 | 159 | 4 |
| **38** | 6 | Homogen | 0.5 | 96.0 | 264 | 55 |
| **39** | 16 | Heterogen | 0.5 | 89.8 | 542 | 62 |
| **40** | 16 | Heterogen | 2.0 | 98.9 | 149 | 67 |
| **41** | 16 (recycliert) | Heterogen | 2.0 | 100 | 151 | 66 |

**Substrat : Phenylglyoxylsäuremethylester**

| **Biespeil** | **Katalysator aus Beispiel** | **Temperatur [°C]** | **Reaktionsdauer [%]** | **Umsatz [%]** | **ce [%]** |
|---|---|---|---|---|---|
| **42** | 3 | 40 | 2 | 82,0 | 0 |
| | | | | | |
| **43** | 2 | 40 | 2 | 98,9 | 93,3 |
| | | | | | |
| **44** | 4 | 40 | 2 | 98,3 | 89,1 |

## Patentansprüche

1. Substanzen enthaltend zumindest
• ein mikro-, meso- oder makroporöses Trägermaterial und
• daran und/oder darin adsorbierte Verbindungen der Formel (I) 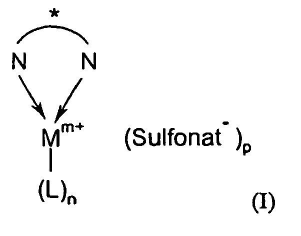 in der 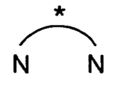 für eine enantiomerenangereicherte chirale Stickstoffverbindung steht,
(M ^{m+}) für ein Metall mit der Wertigkeit m steht
L für einen anionischen oder neutralen Liganden steht
(Sulfonat⁻) für das Anion einer Sulfonsäure steht und
p für eins oder zwei steht und
n für eins, zwei, drei oder vier steht,
wobei die Auflage gilt, dass m-p-[Anzahl der anionischen Liganden] = 0 ist.

2. Substanzen nach Anspruch 1, **dadurch gekennzeichnet, dass** die Trägermaterialien eine Porengröße von 15 bis 250 Å aufweisen.

3. Substanzen nach mindestens einem der Ansprüche 1 und 2, **dadurch gekennzeichnet, dass** die **Trägermaterialien** Silica-Gele oder Zeolithe vom Typ MOR, X, Y, MCM, ZSM, FAU, MFI, L, BEA, FER, A und SBA, AIPO, MAIPO und SAPO sind, wobei die genannten Zeolithe gegebenenfalls isomorph substituiert sein können.

4. Substanzen nach mindestens einem der Ansprüche 1 bis 3, **dadurch gekennzeichnet, dass** in Formel (I) 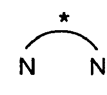 für enantiomerenangereicherte chirale Stickstoffverbindungen der Formel (II) steht 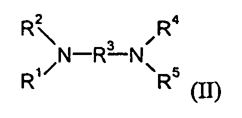 in der
R¹, R², R⁴ und R⁵ jeweils unabhängig voneinander für Wasserstoff, C₁-C₈-Alkyl, C₅-C₁₅₋Arylalkyl oder C₄-C₁₄-Aryl stehen oder NR¹R² und/oder NR⁴R⁵ als Ganzes für einen cyclischen Aminorest mit insgesamt 4 bis 20 Kohlenstoffatomen steht,
R³ für einen divalenten Rest mit 2 bis 30 Kohlenstoffatomen steht oder
R³ und mindestens einer der Reste R¹, R², R⁴, R⁵ zusammen Teil eines cyclischen Aminorestes mit insgesamt 4 bis 20 Kohlenstoffatomen sind.

5. Substanzen nach Anspruch 4, **dadurch gekennzeichnet, dass**
R¹, R², R⁴ und R⁵jeweils unabhängig voneinander für Wasserstoff, C₁-C₈-Alkyl, C₅-C₁₅₋Arylalkyl oder C₄-C₁₄-Aryl stehen oder NR¹R² und/oder NR⁴R⁵ als Ganzes für einen 5- oder 6-gliedrigen monocyclischen Aminorest steht, der am Kohlenstoffgerüst gegebenenfalls einfach, zweifach, dreifach oder vierfach C₁-C₄₋Alkyl substituiert ist und
R³ für einen divalenten Rest steht, der ausgewählt ist aus der Gruppe C₂-C₈-Alkylen, das gegebenenfalls einfach oder zweifach durch C₄-C₁₄-Arylreste weiter substituiert sein kann, C₅-C₁₅-Arylalkylen, C₄-C₁₄-Arylen oder Bis-(C₄-C₁₄-arylen) oder
R³und einer der Reste R¹, R², R⁴ und R⁵ zusammen Teil eines 5- oder 6-gliedrigen monocyclischen Aminorestes sind, der am Kohlenstoffgerüst gegebenenfalls zusätzlich einfach, zweifach, dreifach oder vierfach durch C₁-C₄-Alkyl substituiert ist.

6. Substanzen nach mindestens einem der Ansprüche 1 bis 5, **dadurch gekennzeichnet, dass** in Formel (I)
(M^{m+}) für Cobalt in den formalen Oxidationsstufen 0, +2 und +3, Rhodium und Iridium in den formalen Oxidationsstufen +1 und +3, Nickel, Palladium und Platin in den formalen Oxidationsstufen 0 und +2 oder Ruthenium in der formalen Oxidationsstufe +2 steht.

7. Substanzen nach mindestens einem der Ansprüche 1 bis 6, **dadurch gekennzeichnet, dass** in Formel (I)
L für folgende Ligandentypen steht: Monoolefine, Diolefine, Nitrile, Aromaten sowie anionische Liganden.

8. Substanzen nach mindestens einem der Ansprüche 1 bis 7, **dadurch gekennzeichnet, dass** in Formel (I)
(Sulfonat⁻) für Salze des Typs R₆SO₃⁻ steht, wobei R⁶ für C₁-C₁₂-Alkyl, C₁-C₂₀₋Halogenalkyl, C₄-C₁₄-Aryl oder C₅-C₁₅-Arylalkyl steht.

9. Substanzen nach mindestens einem der Ansprüche 1 bis 8, **dadurch gekennzeichnet, dass** Verbindungen der Formel (I) solche der Formeln (Ia), (Ib), (Ic), (Id). (e) und (If) sind 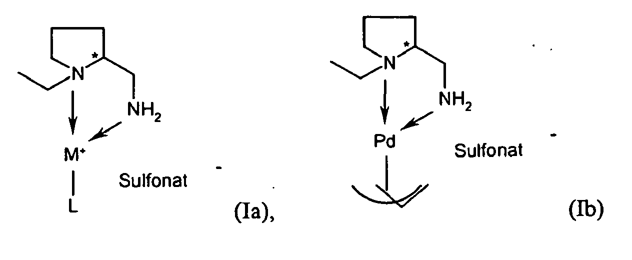 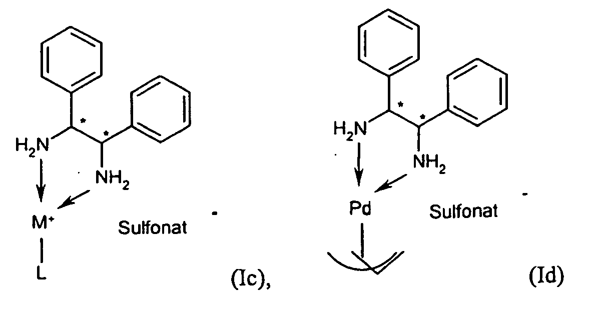 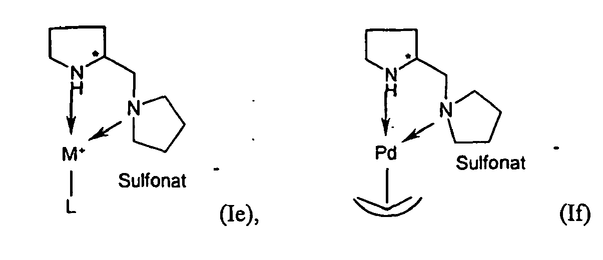 in denen jeweils
* ein stereogenes Zentrum markiert, das entweder R oder S konfiguriert ist, wobei die Auflage gilt, dass Mesoformen ausgeschlossen sind (Verbindungen der Formel (Ic) und (Id))
M⁺ für Rhodium^{I} oder Iridium^{I} steht und
L für cod oder nbd steht und
Sulfonat⁻ für Trifluormethansulfonat, Mesylat oder Nonafluorbutansulfonat steht.

10. Verbindungen der Formel (I) mit der in Anspruch 1 genannten Bedeutung, wobei folgende Verbindungen ausgenommen sind:
[Rh(cod)((S)-2-aminomethyl-1-ethylpyrrolidin)]OTf und [Rh(cod)((1R,2R)-1,2-Diphenylethylendiamin)]OTf.

11. Verwendung von Substanzen nach mindestens einem der Ansprüche 1 bis 9 als Katalysator für asymmetrische Reaktionen.

12. Katalysatoren enthaltend Substanzen nach mindestens einem der Ansprüche 1 bis 9.

13. Verfahren zur katalytischen Herstellung von enantiomerenangereicherten Verbindungen ümfasst, das **dadurch gekennzeichnet ist, dass** Katalysatoren nach Anspruch 12 eingesetzt werden.

14. Verfahren nach Anspruch 13, **dadurch gekennzeichnet, dass** Verfahren zur katalytischen Herstellung von enantiomerenangereicherten Verbindungen asymmetrische Hydrierungen sind.

15. Verfahren nach Anspruch 14, **dadurch gekennzeichnet, dass** asymmetrische Hydrierungen Hydrierungen von prochiralen C=C-Bindungen, C=O-Bindungen und C=N-Bindungen sind.

16. Verfahren nach Anspruch 15, **dadurch gekennzeichnet, dass** Hydrierungen von prochiralen C=O-Bindungen Hydrierungen α- und β-Ketocarbonsäureestern sind.

17. Verfahren nach Anspruch 16, **dadurch gekennzeichnet, dass** α- und β-Ketocarbonsäureester solche der Formel (IV) sind 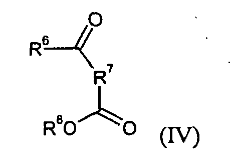 in der
R⁶ und R⁸ jeweils unabhängig voneinander für C₁-C₁₂-Alkyl, C₁-C₁₂-Halogenalkyl, C₅-C₁₅₋Arylalkyl oder C₄-C₁₄-Aryl stehen und
R⁷ fehlt oder für 1,1-(C₁-C₄-Alkylen) steht.

18. Verfahren nach mindestens einem der Ansprüche 14 bis 17, **dadurch gekennzeichnet, dass** für asymmetrische Hydrierungen die Reaktionstemperatur 0 bis 200°C und der Wasserstoffpartialdruck 0,1 bis 200 bar beträgt.

19. Verfahren nach mindestens einem der Ansprüche 14 bis 18, **dadurch gekennzeichnet, dass** für asymmetrische Hydrierungen als aliphatische oder aromatische, gegebenenfalls halogenierte Kohlenwasserstoffe, Ether und/oder Alkohole eingesetzt werden.

20. Verfahren nach mindestens einem der Ansprüche 13 bis 19, **dadurch gekennzeichnet, dass** das Gewichtsverhältnis von Katalysatoren nach Anspruch 12 zu Substrat 1:1 bis 1:10000 beträgt.

## Claims

1. Substances comprising at least
• one micro-, meso- or macroporous support material and
• compounds, adsorbed thereon or therein, of the formula (I) 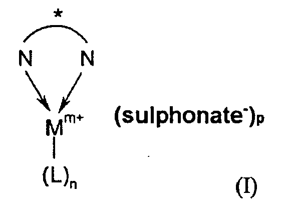
where 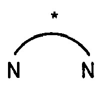 is an enantiomerically enriched chiral nitrogen compound,
(M ^{m+}) is a metal having valency m
L is an anionic or uncharged ligand
(sulphonate⁻) is the anion of a sulphonic acid and
p is one or two and
n is one, two, three or four,
with the proviso that m-p-[number of anionic ligands] = 0.

2. Substances according to Claim 1, **characterized in that** the support materials have a pore size of 15 to 250 Å.

3. Substances according to at least one of Claims 1 and 2, **characterized in that** the support materials are silica gels or zeolites of the MOR, X, Y, MCM, ZSM, FAU, MFI, L, BEA, FER, A and SBA, AlPO, MA1PO and SAPO type, and the zeolites mentioned may optionally be isomorphically substituted.

4. Substances according to at least one of Claims 1 to 3, **characterized in that**, in formula (I), 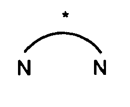 is enantiomerically enriched chiral nitrogen compounds of the formula (II) 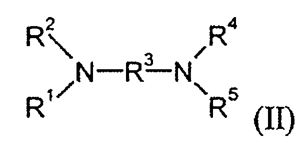 where
R¹, R², R⁴ and R⁵ are each independently hydrogen, C₁-C₈-alkyl, C₅-C₁₅₋arylalkyl or C₄-C₁₄-aryl or NR¹R² and/or NR⁴R⁵ as a whole is a cyclic amino radical having a total of 4 to 20 carbon atoms,
R³ is a divalent radical having 2 to 30 carbon atoms or
R³ and at least one of the radicals R¹, R², R⁴ and R⁵ together are part of a cyclic amino radical having a total of 4 to 20 carbon atoms.

5. Substances according to Claim 4, **characterized in that**
R¹, R², R⁴ and R⁵ are each independently hydrogen, C₁-C₈-alkyl, C₅-C₁₅₋arylalkyl or C₄-C₁₄-aryl or NR¹R² and/or NR⁴R⁵ as a whole is a 5- or 6-membered monocyclic amino radical which is optionally mono-, di-, tri- or tetrasubstituted on the carbon framework by C₁-C₄-alkyl and
R³ is a divalent radical which is selected from the group of C₂-C₈₋alkylene which may optionally be further mono- or diubstituted by C₄-C₁₄-aryl radicals, C₅-C₁₅-arylalkylene, C₄-C₁₄-arylene or bis(C₄₋C₁₄-arylene) or
R³ and one of the radicals R¹, R², R⁴ and R⁵ together are part of a 5- or 6-membered monocyclic amino radical which is optionally additionally mono-, di-, tri- or tetrasubstituted on the carbon framework by C₁-C₄-alkyl.

6. Substances according to at least one of Claims 1 to 5, **characterized in that**, in formula (I),
(M ^{m+}) is cobalt in the formal oxidation states 0, +2 and +3, rhodium and iridium in the formal oxidation states +1 and +3, nickel, palladium and platinum in the formal oxidation states 0 and +2 or ruthenium in the formal oxidation state +2.

7. Substances according to at least one of Claims 1 to 6, **characterized in that**, in formula (I),
L is the following types of ligand: monoolefins, diolefins, nitriles, aromatics and also anionic ligands.

8. Substances according to at least one of Claims 1 to 7, **characterized in that**, in formula (I),
(sulphonate-) is salts of the type R₆SO₃⁻ where R⁶ is C₁-C₁₂-alkyl, C₁-C₂₀₋haloalkyl, C₄-C₁₄-aryl or C₅-C₁₅-arylalkyl.

9. Substances according to at least one of Claims 1 to 8, **characterized in that** compounds of the formula (I) are those of the formulae (Ia), (Ib), (Ic), (Id), (Ie) and (If) 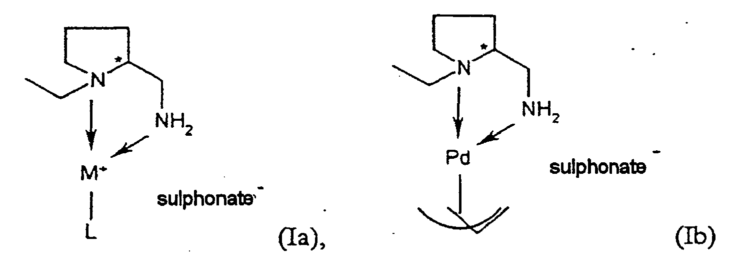 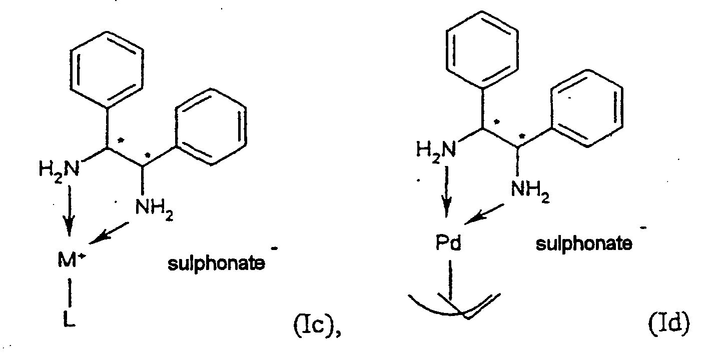 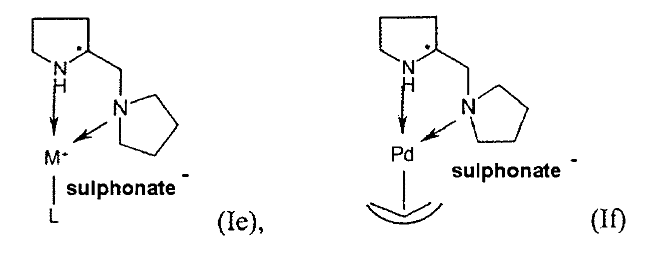 where, in each case,
* marks a stereogenic centre which is either R- or S-configured, with the proviso that mesoforms are excluded (compounds of the formula (Ic) and (Id))
M⁺ is rhodium^{I} or iridium^{I} and
L is cod or nbd and
sulphonate⁻ is trifluoromethanesulphonate, mesylate or nonafluorobutane-sulphonate.

10. Compounds of the formula (I) as defined in Claim 1, with the exception of the following compounds:
[Rh(cod)((S)-2-aminomethyl-1-ethylpyrrolidine)]OTf and
[Rh(cod)((1R,2R)-1,2-diphenylethylenediamine)]OTf.

11. Use of substances according to at least one of Claims 1 to 9 as catalyst for asymmetric reactions.

12. Catalysts comprising substances according to at least one of Claims 1 to 9.

13. Process for catalytically preparing enantiomerically enriched compounds, which is **characterized in that** catalysts according to Claim 12 are used.

14. Process according to Claim 13, **characterized in that** processes for catalytically preparing enantiomerically enriched compounds are asymmetric hydrogenations.

15. Process according to Claim 14, **characterized in that** asymmetric hydrogenations are hydrogenations of prochiral C=C bonds, C=O bonds and C=N bonds.

16. Process according to Claim 15, **characterized in that** hydrogenations of prochiral C=O bonds are hydrogenations of α- and β-keto carboxylic esters.

17. Process according to Claim 16, **characterized in that** α- and β-keto carboxylic esters are those of the formula (IV) 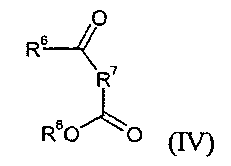 where
R⁶ and R⁸ are each independently C₁-C₁₂-alkyl, C₁-C₁₂-haloalkyl C₅₋C₁₅-arylalkyl or C₄-C₁₄-aryl and
R⁷ is absent or is 1,1-(C₁-C₄-alkylene).

18. Process according to at least one of Claims 14 to 17, **characterized in that** the reaction temperature for asymmetric hydrogenations is 0 to 200°C and the partial hydrogen pressure is 0.1 to 200 bar.

19. Process according to at least one of Claims 14 to 18, **characterized in that** the solvents used for asymmetric hydrogenations are aliphatic or aromatic, optionally halogenated hydrocarbons, ethers and/or alcohols.

20. Process according to at least one of Claims 13 to 19, **characterized in that** the weight ratio of catalysts according to Claim 12 to substrate is 1:1 to 1:10 000.

## Revendications

1. Substances contenant au moins
• une matière de support micro-, méso- ou macroporeuse et
• adsorbés sur et/ou dans celle-ci, des composés de formule (I) 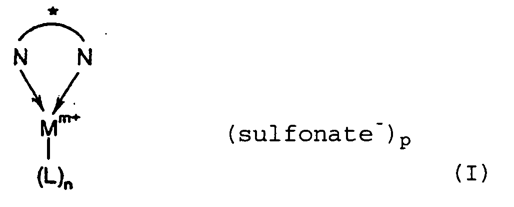
dans laquelle 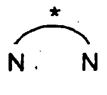 représente un composé azoté chiral enrichi en énantiomère,
(M^{m+}) représente un métal ayant la valence m,
L représente un ligand anionique ou neutre,
(sulfonate⁻) représente l'anion d'un acide sulfonique et
p représente un ou deux et
m représente un, deux, trois ou quatre,
étant entendu que
m-p-[nombre des ligands anioniques] = 0.

2. Substances selon la revendication 1, **caractérisées en ce que** les matières de support ont une taille de pore de 15 à 250 Å.

3. Substances selon au moins l'une des revendications 1 et 2, **caractérisées en ce que** les matières de support sont des gels de silice ou des zéolithes du type MOR, X, Y, MCM, ZSM, FAU, MFI, L, BEA, FER, A et SBA, AlPO, MAlPO et SAPO, les zéolithes citées pouvant éventuellement être substituées de façon isomorphe.

4. Substances selon au moins l'une des revendications 1 à 3, **caractérisées en ce que** dans la formule (I) 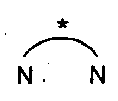 représente des composés azotés chiraux enrichis en énantiomères, de formule (II) 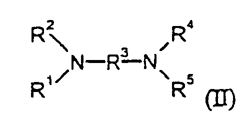 dans laquelle
R¹, R² R⁴ et R⁵ représentent chacun, indépendamment les uns des autres, un atome d'hydrogène, un groupe alkyle en C₁-C₈, arylalkyle en C₅-C₁₅ ou aryle en C₄-C₁₄ ou NR¹R² et/ou NR⁴R⁵ représentent chacun dans son ensemble un radical amino cyclique ayant au total de 4 à 20 atomes de carbone,
R³ représente un radical divalent ayant de 2 à 30 atomes de carbone ou
R³ et au moins un des radicaux R¹, R², R⁴, R⁵ font ensemble partie d'un radical amino cyclique ayant au total de 4 à 20 atomes de carbone.

5. Substances selon la revendication 4, **caractérisées en ce que**
R¹, R², R⁴ et R⁵ représentent chacun, indépendamment les uns des autres, un atome d'hydrogène, un groupe alkyle en C₁-C₈, arylalkyle en C₅-C₁₅ ou aryle en C₄-C₁₄, ou NR¹R² et/ou NR⁴R⁵ représentent chacun dans son ensemble un radical amino monocyclique à 5 ou 6 chaînons, qui est éventuellement une, deux, trois ou quatre fois substitué sur le squelette carboné par alkyle en C₁-C₄, et
R³ représente un radical divalent qui est choisi dans l'ensemble constitué par un groupe alkylène en C₂-C₈ qui peut éventuellement être encore mono- ou disubstitué par des radicaux aryle en C₄-C₁₄, un groupe arylalkylène en C₅-C₁₅, arylène en C₄-C₁₄ ou bis [arylène (C₄-C₁₄)] ou
R³ et l'un des radicaux R¹ R², R⁴ et R⁵ font ensemble partie d'un radical amino monocyclique à 5 ou 6 chaînons qui est éventuellement en plus substitué une, deux, trois ou quatre fois sur le squelette carboné par alkyle en C₁-C₄.

6. Substances selon au moins l'une des revendications 1 à 5, **caractérisées en ce que** dans la formule (I)
(M^{m+}) représente le cobalt aux degrés d'oxydation de par la formule 0, +2 et +3, le rhodium et l'iridium aux degrés d'oxydation de par la formule +1 et +3, le nickel, le palladium et le platine aux degrés d'oxydation de par la formule 0 et +2 ou le ruthénium au degré d'oxydation de par la formule +2.

7. Substances selon au moins l'une des revendications 1 à 6, **caractérisées en ce que** dans la formule (I)
L représente les types de ligands suivants : mono-oléfines, dioléfines, nitriles, composés aromatiques ainsi que ligands anioniques.

8. Substances selon au moins l'une des revendications 1 à 7, **caractérisées en ce que** dans la formule (I)
(sulfonate⁻) représente des sels du type R₆SO₃⁻, R⁶ représentant un groupe alkyle en C₁-C₁₂, halogénoalkyle en C₁-C₂₀, aryle en C₄-C₁₄ ou arylalkyle en C₅-C₁₅.

9. Substances selon au moins l'une des revendications 1 à 8, **caractérisées en ce que** les composés de formule (I) sont ceux de formules (Ia), (Ib), (Ic), (Id), (Ie) et (If), 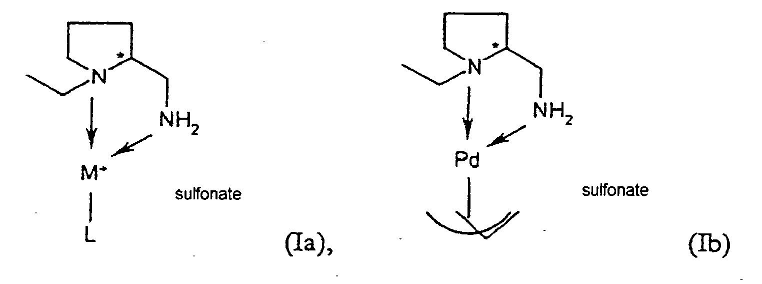 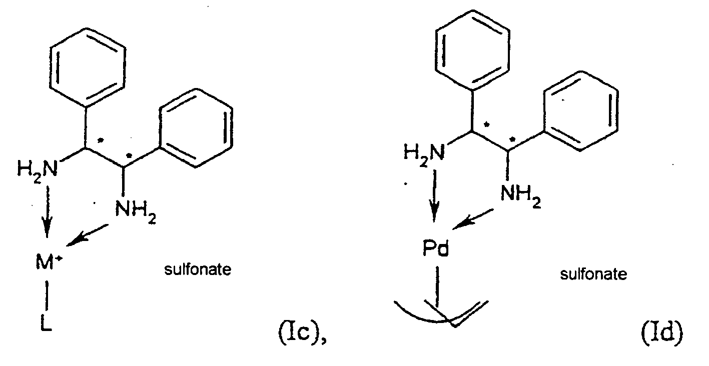 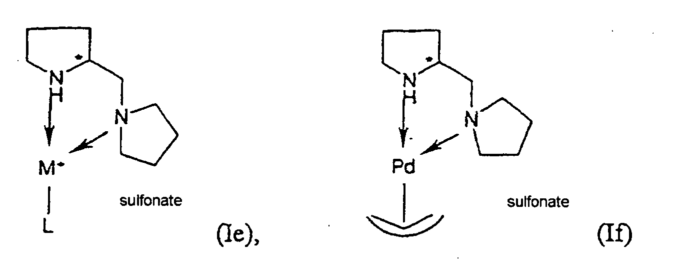 dans lesquelles
* désigne un centre stéréogène qui est en configuration soit R, soit S, étant entendu que les mésoformes sont exclues (composés de formule (Ic) et (Id))
M⁺ représente le rhodium¹ ou l'iridium¹ et
L représente cod ou nbd et
Sulfonate⁻ représente l'ion trifluorométhane-sulfonate, mésylate ou nonafluorobutanesulfonate.

10. Composés de formule (I) avec la signification donnée dans la revendication 1, à l'exclusion des composés suivants :
[Rh (cod) ((S) -2-aminométhyl-1-éthylpyrrolidine)] OTf et [Rh (cod) ((1R,2R)-1,2-diphényléthylènediamine)]-OTf.

11. Utilisation de substances selon au moins une des revendications 1 à 9, en tant que catalyseur pour des réactions asymétriques.

12. Catalyseurs contenant des substances selon au moins l'une revendications 1 à 9.

13. Procédé pour la préparation catalytique de composés enrichis en énantiomères, **caractérisé en ce qu'**on utilise des catalyseurs selon la revendication 12.

14. Procédé selon la revendication 13, **caractérisé en ce que** les procédés pour la préparation catalytique de composés enrichis en énantiomères sont des hydrogénations asymétriques.

15. Procédé selon la revendication 14, **caractérisé en ce que** les hydrogénations asymétriques sont des hydrogénations de liaisons C=C, de liaisons C=O et de liaisons C=N prochirales.

16. Procédé selon la revendication 15, **caractérisé en ce que** les hydrogénations de liaisons C=O prochirales sont des hydrogénations d'esters d'acides α- et β-cétocarboxyliques.

17. Procédé selon la revendication 16, **caractérisé en ce que** les esters d'acides α- et β-cétocarboxyliques sont ceux de formule (IV) 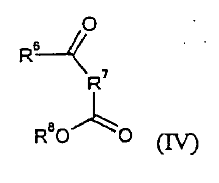 dans laquelle
R⁶ et R⁸ représentent chacun, indépendamment l'un de l'autre, un groupe alkyle en C₁-C₁₂, halogénoalkyle en C₁-C₁₂, arylalkyle en C₅-C₁₅ ou aryle en C₄-C₁₄ et
R⁷ est absent ou représente un groupe 1,1-(alkylène en C₁-C₄) .

18. Procédé selon au moins l'une des revendication 14 à 17, **caractérisé en ce que** pour des hydrogénations asymétriques la température de réaction est de 0 à 200 °C et la pression partielle d'hydrogène est de 0,1 à 200 bars.

19. Procédé selon au moins l'une des revendication 14 à 18, **caractérisé en ce que** pour des hydrogénations asymétriques on utilise des éthers et/ou des alcools en tant qu'hydrocarbures aliphatiques ou aromatiques, éventuellement halogénés.

20. Procédé selon au moins l'une des revendication 13 à 19, **caractérisé en ce que** le rapport pondéral des catalyseurs selon la revendication 12 au substrat va de 1:1 à 1:10000.
